(19) Europäisches Patentamt
European Patent Office
Office européen des brevets

(11) Veröffentlichungsnummer: **0 119 446 A2**

# (12) EUROPÄISCHE PATENTANMELDUNG

(21) Anmeldenummer: **84101377.4**

(22) Anmeldetag: **10.02.84**

(51) Int. Cl.³: **C 07 C 149/36**, C 07 C 147/10, C 07 C 149/42, C 07 C 91/40, C 07 D 209/02, C 07 D 295/12, C 07 F 7/10

(30) Priorität: **22.02.83 DE 3306160**

(43) Veröffentlichungstag der Anmeldung: **26.09.84 Patentblatt 84/39**

(84) Benannte Vertragsstaaten: **AT BE CH DE FR GB IT LI LU NL SE**

(71) Anmelder: **BAYER AG, Konzernverwaltung RP Patentabteilung, D-5090 Leverkusen 1 Bayerwerk (DE)**

(72) Erfinder: **Böshagen, Horst, Dr., Wiesenstrasse 4, D-5657 Haan (DE)**
Erfinder: **Stoltefuss, Jürgen, Dipl.-Ing., Parkstrasse 20, D-5657 Haan (DE)**
Erfinder: **Berschauer, Friedrich, Dr., Claudiusweg 9, D-5600 Wuppertal 1 (DE)**
Erfinder: **De Jong, Anno, Dr., Stockmannsmühle 46, D-5600 Wuppertal 1 (DE)**
Erfinder: **Scheer, Martin, Dr., Herberts-Katenberg 7, D-5600 Wuppertal 1 (DE)**

(54) **Wachstumsfördernde Aminophenylethylamin-Derivate.**

(57) Die Erfindung betrifft Phenylethylamin-Derivate der allgemeinen Formel I

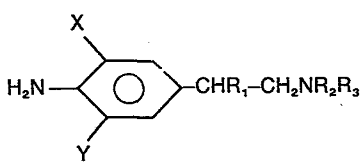

in der die Reste $R_1$, $R_2$, $R_3$, X und Y die in der Beschreibung angegebene Bedeutung haben, und deren Verwendung als wachstumsfördernde Zusätze in der Tiernahrung.

EP 0 119 446 A2

BAYER AKTIENGESELLSCHAFT 5090 Leverkusen, Bayerwerk
Konzernverwaltung RP
Patentabteilung Ad/bc/c

# Wachstumsfördernde Aminophenylethylamin-Derivate

Die Erfindung betrifft Phenylethylamin-Derivate und deren Verwendung in der Tiernahrung zur Wachstumssteigerung und zur Verbesserung des Fleisch/Fett-Verhältnisses.

Die Verwendung von Futtermittelzusätzen zur Erzielung höherer Gewichtszuwächse und verbesserter Futterausnutzung wird in der Tiernahrung insbesondere bei der Mast von Schweinen, Rindern und Geflügel bereits weitgehend praktiziert.

Es wurde nun gefunden, daß Phenylethylamin-Derivate der allgemeinen Formel (I)

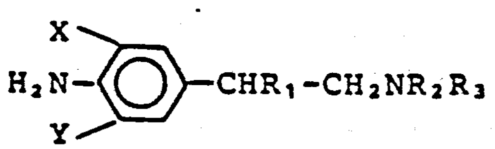

(I)

in der

X und Y gleich oder verschieden sind und für Wasserstoff oder Halogen stehen,

$R_1$ für OH oder einen Rest 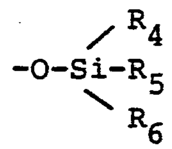 steht,

$R_2$ · Wasserstoff oder einen geradkettigen, verzweigten, gesättigten oder ungesättigten Alkylrest mit bis zu 6 C-Atomen bedeutet mit der Maßgabe, daß $R_2$ nur dann für einen Alkylrest stehen kann, wenn $R_3$ $-SiR_4R_5R_6$ bedeutet,

$R_3$ einen geradkettigen, verzweigten, gesättigten oder ungesättigten Alkylrest, der durch Halogen, bevorzugt Chlor und/oder Fluor substituiert ist, einen Rest $-SiR_4R_5R_6$, einen Phenylrest, der gegebenenfalls durch Halogen, Hydroxy, Alkyl, Aryl, Alkoxy, Alkylthio, gegebenenfalls substituiertes Phenoxy oder Phenylthio, Cyano oder Trifluormethyl substituiert sein kann, einen heterocyclischen Rest oder zusammen mit $R_2$ einen stickstoffhaltigen, gesättigten heterocyclischen Rest wie z.B. Azabicyclononan, substituiertes Azabicyclononan, Azabicyclooctan und ähnliche bedeutet und

$R_4, R_5, R_6$ einen geradkettigen oder verzweigten Alkylrest bedeuten, und deren physiologisch verträglichen Salze eine ausgezeichnete wachstumsfördernde Wirkung besitzen.

Die Substanzen bewirken außerdem eine Verbesserung der Futterverwertung und eine Verschiebung des Fleisch/Fett-Verhältnisses zugunsten von Fleisch.

Bevorzugte Verbindungen sind Phenylethylamin-Derivate der allgemeinen Formel (I), in der

X und Y für Chlor stehen,

$R_1$ $O-SiR_4R_5R_6$,

$R_2$ einen gesättigten oder ungesättigten, verzweigten oder unverzweigten Alkylrest mit bis zu 6 C-Atomen und

$R_3$ den Rest $-SiR_4R_5R_6$ bedeutet,

oder Verbindungen der Formel (I), in denen

X und Y Chlor,

$R_1$ OH,

$R_2$ Wasserstoff und

$R_3$ entweder einen durch ein oder mehrere Chlor- oder Fluoratome substituierten geradkettigen oder verzweigten, gesättigten oder ungesättigten $C_1$-$C_6$-Alkylrest oder einen Phenylrest, der gegebenenfalls durch Halogen, Hydroxy, $C_1$-$C_4$-Alkyl, $C_1$-$C_4$-Alkoxy, $C_6$-$C_{10}$-Aryl, $C_1$-$C_4$-Alkylthio oder durch gegebenenfalls substituiertes Phenoxy oder Phenylthio substituiert sein kann, bedeutet,

sowie deren physiologisch verträglichen Salze.

Besonders bevorzugte Verbindungen sind Phenylethylamin-Derivate der Formel (I), in der

X und Y für Chlor,

$R_1$ für OH,

$R_2$ für Wasserstoff und

$R_3$ für eine durch ein oder mehrere $CF_3S$, $CF_3SO_2$, $C_1$-$C_4$-Alkyl, Halogen oder Phenyl-Reste substituierte Phenoxyphenyl- oder Phenylthiophenyl-Gruppe steht,

sowie deren physiologisch verträglichen Salze.

Herstellungsverfahren für Verbindungen dieser Art sind bekannt und werden z.B. in der DE-OS 1 543 928 beschrieben. An pharmakologischen Wirkungen stand bisher die ausgeprägte Kreislaufwirkung dieser Stoffe im Vordergrund. (J. Keck et al., Arzneim.-Forsch. 22, 861-869 (1972)).

Die erfindungsgemäß verwendeten Phenylethylamin-Derivate sind in der Literatur nicht beschrieben. Ein weiterer Gegenstand der Erfindung sind demnach neue Phenylethylamin-Derivate der Formel (I), in der

X und Y gleich oder verschieden sind und für Wasserstoff oder Halogen stehen,

$R_1$ für OH oder einen Rest $-O-\underset{R_8}{\overset{R_6}{Si}}-R_7$ steht,

$R_2$ Wasserstoff oder einen geradkettigen, verzweigten, gesättigten oder ungesättigten Alkylrest mit bis zu 6 C-Atomen bedeutet, mit der Maßgabe, daß $R_4$ nur dann für einen Alkylrest stehen kann, wenn $R_2$ $-SiR_4R_5R_6$ bedeutet,

$R_3$ einen geradkettigen, verzweigten, gesättigten oder ungesättigten Alkylrest, der durch Halogen, bevorzugt Chlor oder/und Fluor substituiert ist, einen Rest $-SiR_4R_5R_6$, einen Phenylrest, der gegebenenfalls durch Halogen, Hydroxy, Alkyl, Aryl, Alkoxy, Alkylthio, gegebenenfalls substituiertes Phenoxy oder Phenylthio, Cyano oder Trifluormethyl substituiert sein kann, einen heterocyclischen Rest oder

$R_2$ mit $R_3$ zusammen einen stickstoffhaltigen heterocyclischen Rest wie Piperazin, Azabicyclononan oder Azabicyclooctan bedeutet,

und

$R_4$,$R_5$,$R_6$ einen geradkettigen oder verzweigten Alkylrest bedeuten.

Erfindungsgemäß erhält man die neuen Verbindungen nach folgenden Verfahren:

a) Reduktion einer Verbindung der allgemeinen Formel (II)

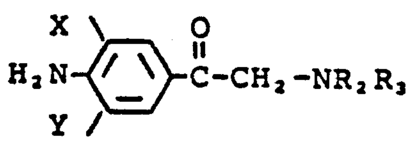

(II)

in der
X,Y,$R_1$ und $R_2$ die oben angegebene Bedeutung haben,

b) Verbindungen, in denen

$R_1$ für den Rest $-O-SiR_4R_5R_6$,
$R_2$ für einen geradkettigen, verzweigten, gesättigten oder ungesättigten Alkylrest mit bis zu 6 C-Atomen und
$R_3$ für den Rest $-SiR_4R_5R_6$ steht,

erhält man, indem man Verbindungen der allgemeinen Formel (III)

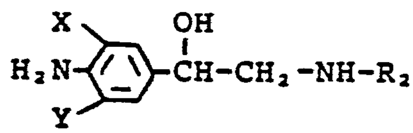

(III)

in der
X,Y und $R_2$ die oben angegebene Bedeutung haben,

mit geeigneten Silylierungsmitteln der Formel (IV)

$$R_4R_5R_6\ Si{-}Z \qquad (IV)$$

in der
Z Halogen, $-O-SO_2-CF_3$, $-O-SiR_4R_5R_6$ oder $-O-SO_2-O-SiR_4R_5R_6$ bedeutet,

umsetzt.

Die Ausgangsverbindungen der Formeln (II) und (III) sind entweder bekannt oder können nach bekannten Methoden hergestellt werden (vgl. z.B. R.E. Lutz and Co-workers, J.Org.Chem. 12, 617-703 (1974)).

Die Wirkstoffe können in allen Bereichen der Tierzucht als Mittel zur Förderung und Beschleunigung des Wachstums und zur Verbesserung der Futterverwertung bei gesunden und kranken Tieren verwendet werden.

Die Wirksamkeit der Wirkstoffe ist hierbei weitgehend unabhängig von der Art und dem Geschlecht der Tiere. Besonders wertvoll erweisen sich die Wirkstoffe bei der Aufzucht und Haltung von Jung- und Masttieren.

Als Tiere, bei denen die Wirkstoffe zur Förderung und Beschleunigung des Wachstums und zur Verbesserung der Futterverwertung eingesetzt werden können, seien beispielsweise folgende Nutz- und Ziertiere genannt:

Warmblüter wie Rinder, Schweine, Pferde, Schafe, Ziegen, Katzen, Hunde, Kaninchen, Pelztiere, z.B. Nerze, und Chinchilla, Geflügel, z.B. Hühner, Gänse, Enten, Truthähne, Tauben, Papageien und Kanarienvögel und Kaltblüter wie Fische, z.B. Karpfen und Reptilien, z.B. Schlangen.

Die Mengen der Wirkstoffe, die den Tieren zur Erreichung des gewünschten Effektes verabreicht wird, kann wegen der günstigen Eigenschaften der Wirkstoffe weitgehend variiert werden. Sie liegt vorzugsweise bei etwa 0,01 bis 50, insbesondere 0,1 bis 10 mg/kg Körpergewicht pro Tag. Die Dauer der Verabreichung kann von wenigen Stunden oder Tagen bis zu mehreren Jahren betragen. Die passende Menge des Wirkstoffs sowie die passende Dauer der Verabreichung hängen insbesondere von der Art, dem Alter, dem Geschlecht, dem Gesundheitszustand und der Art der Haltung und Fütterung der Tiere ab und sind durch jeden Fachmann leicht zu ermitteln.

Die Wirkstoffe werden den Tieren nach den üblichen Methoden verabreicht. Die Art der Verabreichung hängt insbesondere von der Art, dem Verhalten und dem Gesundheitszustand der Tiere ab. So kann die Verabreichung einmal oder mehrmals täglich in regelmäßigen oder unregelmäßigen Abständen oral oder parenteral erfolgen.

Aus Zweckmäßigkeitsgründen ist in den meisten Fällen eine orale Verabreichung, insbesondere im Rhythmus der Nahrungs- und/oder Getränkeaufnahme der Tiere, vorzuziehen.

Die Wirkstoffe können als reine Stoffmischung oder in formulierter Form, also in Mischung mit nicht-toxischen inerten Trägerstoffen beliebiger Art, z.B. mit Trägerstoffen und in Formulierungen, wie sie bei nutritiven Zubereitungen üblich sind, verabreicht werden.

Die Wirkstoffe werden gegebenenfalls in formulierter Form zusammen mit pharmazeutischen Wirkstoffen, Mineralsalzen, Spurenelementen, Vitaminen, Eiweißstoffen, Fetten, Farbstoffen und/oder Geschmacksstoffen in geeigneter Form verabreicht.

Empfehlenswert ist die orale Verabreichung zusammen mit dem Futter und/oder Trinkwasser, wobei je nach Bedarf der Wirkstoff der Gesamtmenge oder nur Teilen des Futters und/oder des Trinkwassers zugegeben wird.

Die Wirkstoffe werden nach üblichen Methoden durch einfaches Mischen als reine Stoffmischung, vorzugsweise in fein verteilter Form oder in formulierter Form in Mischung mit eßbaren nicht-toxischen Trägerstoffen, gegebenenfalls in Form eines Praemix oder eines Futterkonzentrates, dem Futter und/oder Trinkwasser beigefügt.

Das Futter und/oder Trinkwasser kann beispielsweise die Wirkstoffe in einer Gewichtskonzentration von etwa 0,01

bis 50, insbesondere 0,1 bis 10 ppm, enthalten. Die optimale Höhe der Konzentration der Wirkstoffe in dem Futter und/oder Trinkwasser ist insbesondere abhängig von der Menge der Futter- und/oder Trinkwasseraufnahme der Tiere und kann durch jeden Fachmann leicht ermittelt werden.

Die Art des Futters und seine Zusammensetzung ist hierbei ohne Belang. Es können alle gebräuchlichen oder speziellen Futterzusammensetzungen verwendet werden, die vorzugsweise das übliche, für eine ausgewogene Ernährung notwendige Gleichgewicht aus Energie- und Aufbaustoffen einschließlich Vitaminen und Mineralstoffen enthalten. Das Futter kann sich beispielsweise zusammensetzen aus pflanzlichen Stoffen, z.B. Heu, Rüben, Getreide, Getreidenebenprodukten, tierischen Stoffen, z.B. Fleisch, Fetten, Knochenmehl, Fischprodukten, Vitaminen, z.B. Vitamin A, D-Komplex und B-Komplex, Proteinen, Aminosäuren, z.B. DL-Methionin und anorganischen Stoffen, z.B. Kalk und Kochsalz.

Futterkonzentrate enthalten die Wirkstoffe neben eßbaren Stoffen, z.B. Roggenmehl, Maismehl, Sojabohnenmehl oder Kalk, gegebenenfalls mit weiteren Nähr- und Aufbaustoffen sowie Proteinen, Mineralsalzen und Vitaminen. Sie können nach den üblichen Mischmethoden hergestellt werden.

Vorzugsweise in Praemixen und Futterkonzentraten können die Wirkstoffe gegebenenfalls auch durch ihre Oberfläche bedeckende geeignete Mittel, z.B. mit nicht-toxischen Wachsen oder Gelatine vor Luft, Licht und/oder Feuchtigkeit geschützt werden.

Beispiel für die Zusammensetzung eines Kükenaufzuchtfutters, das einen erfindungsgemäßen Wirkstoff enthält:

200 g Weizen, 340 g Mais, 361 g Sojaschrot, 60 g Rindertalg, 15 g Dicalciumphosphat, 10 g Calciumcarbonat, 4 g jodiertes Kochsalz, 7,5 g Vitamin-Mineral-Mischung und 2,5 g Wirkstoff-Praemix ergeben nach sorgfältigem Mischen 1 kg Futter.

In einem kg Futtermischung sind enthalten:
600 I.E. Vitamin A, 100 I.E. Vitamin $D_3$, 10 mg Vitamin E, 1 mg Vitamin $K_3$, 3 mg Riboflavin, 2 mg Pyridoxin, 20 mcg Vitamin $B_{12}$, 5 mg Calciumpantothenat, 30 mg Nikotinsäure, 200 mg Cholinchlorid, 200 mg Mn $SO_4$ x $H_2O$, 140 mg Zn $SO_4$ x 7 $H_2O$, 100 mg Fe $SO_4$ x 7 $H_2O$ und 20 mg Cu $SO_4$ x 5 $H_2O$.

Der Wirkstoff-Praemix enthält die Wirkstoffe in der gewünschten Menge, z.B. 10 mg und zusätzlich 1 g DL-Methionin sowie so viel Sojabohnenmehl, daß 2,5 g Praemix entstehen.

Beispiel für die Zusammensetzung eines Schweineaufzuchtfutters, das einen erfindungsgemäßen Wirkstoff enthält:

630 g Futtergetreideschrot (zusammengesetzt aus 200 g Mais, 150 g Gerste-, 150 g Hafer- und 130 g Weizenschrot), 80 g Fischmehl, 60 g Sojaschrot, 60 g Tapiokamehl, 38 g Bierhefe, 50 g Vitamin-Mineral-Mischung

für Schweine (Zusammensetzung z.B. wie beim Kükenfutter), 30 g Leinkuchenmehl, 30 g Maiskleberfutter, 10 g Sojaöl, 10 g Zuckerrohrmelasse und 2 g Wirkstoff-Praemix (Zusammensetzung z.B. wie beim Kükenfutter) ergeben nach sorgfältigem Mischen 1 kg Futter.

Die angegebenen Futtergemische sind vorzugsweise zur Aufzucht und Mast von Küken bzw. Schweinen abgestimmt, sie können jedoch in gleicher oder ähnlicher Zusammensetzung auf zur Aufzucht und Mast anderer Tiere verwendet werden.

Mit den erfindungsgemäßen Wirkstoffen wurden mehrere Fütterungs- und Stoffwechseluntersuchungen durchgeführt.

Dabei wurden folgende Ergebnisse erhalten:

## Beispiel 1a

a) Tier-Charakteristik und Futter

| | |
|---|---|
| a 1) Ratten, weiblich | |
| a 2) Anzahl | 18 |
| a 3) Zucht | SPF Wistar, Züchtung Hagemann |
| a 4) Gewicht | 90-150 g |
| a 5) Zustand | gut |
| a 6) Futter | |

Rohnährstoffe *

| | |
|---|---|
| Rohprotein | 19,0 |
| Rohfett | 4,0 |
| Rohfaser | 6,0 |
| Asche | 7,0 |
| Wasser | 13,5 |
| N-freie Extraktst. | 50,5 |

Umsetzbare Energie:

| | |
|---|---|
| Kcal/kg | 3100 |
| KJ/kg | 13000 |

Mineralstoffe *

| | |
|---|---|
| Calcium | 0,9 |
| Phosphor | 0,7 |
| Magnesium | 0,2 |
| Natrium | 0,2 |

Vitamine **

Standard-Diät

| | | |
|---|---|---|
| Vitamin A | 15000 | IE |
| Vitamin $D_3$ | 600 | IE |
| Vitamin E | 75 | mg |
| Vitamin $K_3$ | 3 | mg |
| Vitamin $B_1$ | 18 | mg |
| Vitamin $B_2$ | 12 | mg |
| Vitamin $B_6$ | 9 | mg |
| Vitamin $B_{12}$ | 24 | mcg |
| Nikotinsäure | 36 | mg |
| Pantothensäure | 21 | mg |
| Folsäure | 2 | mg |
| Biotin | 60 | mg |
| Cholin | 600 | mg |
| Vitamin C | 36 | mg |

Aminosäuren *

| | |
|---|---|
| Lysin | 0,9 |
| Methionin + Cystin | 0,6 |
| Phenylalanin+Tyrosin | 1,4 |
| Arginin | 1,1 |
| Histidin | 0,4 |
| Tryptophan | 0,2 |
| Threonin | 0,6 |
| Isoleucin | 0,9 |
| Leucin | 1,3 |
| Valin | 0,9 |

Spurenelemente **

| | |
|---|---|
| Magnan | 75,0 |
| Eisen | 135,0 |
| Kupfer | 13,0 |
| Zink | 70,0 |
| Jod | 0,9 |
| Fluor | 9,0 |

* % in der Diät (Mittelwert)
** mg in 1 kg Diät (Mittelwert)

b) Behandlung der Tiere

Die Tiere wurden 2 Tage an die neuen Haltungsbedingungen adaptiert, wobei generell das Versuchsfutter ohne Wirkstoffzusatz verabreicht wurde. Am dritten Versuchstag wurden die Tiere randomisiert und anschließend die Versuchsgruppen so gebildet, daß sowohl die Mittelwerte als auch die Streuungen in den Körpergewichten zwischen den Gruppen gleich waren. Nach einer 5-tägigen Vorperiode wurde eine 8-tägige Hauptperiode durchgeführt, in denen Futteraufnahme, Zuwachs und Futterverwertung bestimmt wurden.

Es wurden folgende Behandlungen geprüft:

b 1) Negativkontrolle (n = 12)

b 2) 25 ppm (Wirkstoff Beispiel 12) (n = 6)

c) Ergebnis (Futteraufnahme, Wachstum, Futterverwertung) während der gesamten Versuchsperiode (13 Tage)

| | Futteraufnahme (g) | Zuwachs (g) | Futterverwertung (g/g) |
|---|---|---|---|
| c 1) Negativkontrolle | 174 | 35,4 | 5,06 |
| c 2) 25 ppm (Wirkstoff Bsp. 12) | 193 | 51,9 | 3,77 |

Beispiel 1b

a) Tier-Charakteristik und Futter

| | |
|---|---|
| a 1) Ratten, weiblich | |
| a 2) Anzahl | 36 |
| a 3) Zucht | SPF Wistar, Züchtung Hagemann |
| a 4) Gewicht | 90-150 g |
| a 5) Zustand | gut |
| a 6) Futter | wie in Beispiel 1a |

b) Behandlung der Tiere

Die Tiere wurden 2 Tage an die neuen Haltungsbedingungen adaptiert, wobei generell das Versuchsfutter

ohne Wirkstoffzusatz verabreicht wurde. Am dritten Versuchstag wurden die Tiere randomisiert und anschließend die Versuchsgruppen so gebildet, daß sowohl die Mittelwerte als auch die Streuungen in den Körpergewichten zwischen den Gruppen gleich waren. Nach einer 5-tägigen Vorperiode wurde eine 7-tägige Hauptperiode durchgeführt, in denen Futteraufnahme, Zuwachs und Futterverwertung bestimmt wurden.

Es wurden folgende Behandlungen geprüft:

b 1) Negativkontrolle (n = 12)
b 2) 0,2 ppm (Wirkstoff Beispiel 12) (n = 6)
b 3) 10 ppm (Wirkstoff Beispiel 12) (n = 6)

c) Ergebnis (Futteraufnahme, Wachstum, Futterverwertung) während der gesamten Versuchsperiode (12 Tage)

| | Futteraufnahme (g) | Zuwachs (g) | Futterverwertung (g/g) |
|---|---|---|---|
| c 1) Negativkontrolle | 200 | 41,0 | 4,88 |
| c 2) 0,2 ppm (Wirkstoff Bsp. 12) | 217 | 52,5 | 4,13 |
| c 3) 10 ppm (Wirkstoff Bsp. 12) | 204 | 50,7 | 4,03 |

0119446

Beispiel 2a

a) Tier-Charakteristik und Futter

| | |
|---|---|
| a 1) Ratten, weiblich | |
| a 2) Anzahl | 18 |
| a 3) Zucht | SPF Wistar, Züchtung Hagemann |
| a 4) Gewicht | 90-150 g |
| a 5) Zustand | gut |
| a 6) Futter | wie in Beispiel 1 |

b) Behandlung der Tiere

Die Tiere wurden 2 Tage an die neuen Haltungsbedingungen adaptiert, wobei generell das Versuchsfutter ohne Wirkstoffzusatz verabreicht wurde. Am dritten Versuchstag wurden die Tiere randomisiert und anschließend die Versuchsgruppen so gebildet, daß sowohl die Mittelwerte als auch die Streuungen in den Körpergewichten zwischen den Gruppen gleich waren. Nach einer 5-tägigen Vorperiode wurde eine 8-tägige Hauptperiode durchgeführt, in denen Futteraufnahme, Zuwachs und Futterverwertung bestimmt wurden.

Es wurden folgende Behandlungen geprüft:

b 1) Negativkontrolle (n = 12)
b 2) 25 ppm (Wirkstoff Beispiel 12) (n = 6)

c) Ergebnis (Futteraufnahme, Wachstum, Futterverwertung) während der gesamten Versuchsperiode (13 Tage)

| | Futter-aufnahme (g) | Zuwachs (g) | Futter-verwer-tung (g/g) |
|---|---|---|---|
| c 1) Negativkontrolle | 174 | 35,4 | 5,06 |
| c 2) 25 ppm (Wirkstoff Bsp. 1) | 184 | 43,9 | 4,23 |

Beispiel 3a

a) Tier-Charakteristik und Futter

| | |
|---|---|
| a 1) Ratten, weiblich | |
| a 2) Anzahl | 18 |
| a 3) Zucht | SPF Wistar, Züchtung Hagemann |
| a 4) Gewicht | 90-150 g |
| a 5) Zustand | gut |
| a 6) Futter | wie in Beispiel 1 |

b) Behandlung der Tiere

Die Tiere wurden 2 Tage an die neuen Haltungsbedingungen adaptiert, wobei generell das Versuchsfutter ohne Wirkstoffzusatz verabreicht wurde. Am dritten Versuchstag wurden die Tiere randomisiert und anschließend die Versuchsgruppen so gebildet, daß sowohl die Mittelwerte als auch die Streuungen in den

Körpergewichten zwischen den Gruppen gleich waren. Nach einer 5-tägigen Vorperiode wurde eine 8-tägige Hauptperiode durchgeführt, in denen Futteraufnahme, Zuwachs und Futterverwertung bestimmt wurden.

Es wurden folgende Behandlungen geprüft:

b 1) Negativkontrolle (n = 12)
b 2) 25 ppm (Wirkstoff Beispiel 2) (n = 6)

c) Ergebnis (Futteraufnahme, Wachstum, Futterverwertung) während der gesamten Versuchsperiode (13 Tage)

| | Futter-aufnahme (g) | Zuwachs (g) | Futter-verwer-tung (g/g) |
|---|---|---|---|
| c 1) Negativkontrolle | 174 | 35,4 | 5,06 |
| c 2) 25 ppm (Wirkstoff Bsp. 2) | 180 | 37,5 | 4,85 |

Beispiel 3b

a) Tier-Charakteristik und Futter

| | |
|---|---|
| a 1) Ratten, weiblich | |
| a 2) Anzahl | 30 |
| a 3) Zucht | SPF Wistar, Züchtung Hagemann |
| a 4) Gewicht | 90-150 g |
| a 5) Zustand | gut |
| a 6) Futter | wie in Beispiel 1 |

b) Behandlung der Tiere

Die Tiere wurden 2 Tage an die neuen Haltungsbedingungen adaptiert, wobei generell das Versuchsfutter ohne Wirkstoffzusatz verabreicht wurde. Am dritten Versuchstag wurden die Tiere randomisiert und anschließend die Versuchsgruppen so gebildet, daß sowohl die Mittelwerte als auch die Streuungen in den Körpergewichten zwischen den Gruppen gleich waren. Nach einer 7-tägigen Vorperiode wurde eine 7-tägige Hauptperiode durchgeführt, in denen Futteraufnahme, Zuwachs und Futterverwertung bestimmt wurden.

Es wurden folgende Behandlungen geprüft:

b 1) Negativkontrolle (n = 24)
b 2) 25 ppm (Wirkstoff Beispiel 2) (n = 6)

c) Ergebnis (Futteraufnahme, Wachstum, Futterverwertung) während der gesamten Versuchsperiode (14 Tage)

| | Futteraufnahme (g) | Zuwachs (g) | Futterverwertung (g/g) |
|---|---|---|---|
| c 1) Negativkontrolle | 225 | 40,6 | 5,30 |
| c 2) 25 ppm (Wirkstoff Bsp. 2) | 233 | 47,8 | 4,88 |

## Beispiel 4

a) Tier-Charakteristik und Futter

| | |
|---|---|
| a 1) Ratten, weiblich | |
| a 2) Anzahl | 36 |
| a 3) Zucht | SPF Wistar, Züchtung Hagemann |
| a 4) Gewicht | 90-150 g |
| a 5) Zustand | gut |
| a 6) Futter | wie in Beispiel 1 |

b) Behandlung der Tiere

Die Tiere wurden 2 Tage an die neuen Haltungsbedingungen adaptiert, wobei generell das Versuchsfutter ohne Wirkstoffzusatz verabreicht wurde. Am dritten Versuchstag wurden die Tiere randomisiert und anschließend die Versuchsgruppen so gebildet, daß sowohl die Mittelwerte als auch die Streuungen in den Körpergewichten zwischen den Gruppen gleich waren. Nach einer 5-tägigen Vorperiode wurde eine 7-tägige Hauptperiode durchgeführt, in denen Futteraufnahme, Zuwachs und Futterverwertung bestimmt wurden.

Es wurden folgende Behandlungen geprüft:

b 1) Negativkontrolle (n = 12)
b 2) 0,2 ppm (Wirkstoff Beispiel 1) (n = 6)
b 3) 10 ppm (Wirkstoff Beispiel 1) (n = 6)

c) Ergebnis (Futteraufnahme, Wachstum, Futterverwertung) während der gesamten Versuchsperiode (12 Tage)

| | Futteraufnahme (g) | Zuwachs (g) | Futterverwertung (g/g) |
|---|---|---|---|
| c 1) Negativkontrolle | 200 | 41,0 | 4,88 |
| c 2) 0,2 ppm (Wirkstoff Bsp. 1) | 204 | 47,9 | 4,25 |
| c 3) 10 ppm (Wirkstoff Bsp. 1) | 206 | 46,2 | 4,46 |

## Herstellungsbeispiele

### Beispiel 1

1-(4-Amino-3,5-dichlor-phenyl)-2-/3-methyl-4-(4-trifluormethylthio-phenoxy)-phenylamino/-ethanol-hydrochlorid

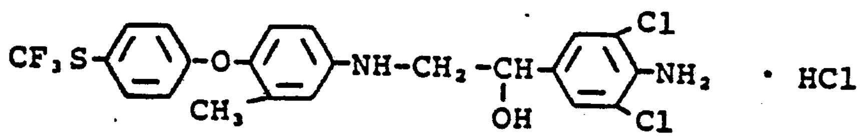

9 g 4-Amino-3,5-dichlor-ω-/2-methyl-4-(4-trifluormethylthiophenoxy)-phenylamino/-acetophenon (hergestellt aus 4-Amino-3,5-dichlor-ω-bromacetophenon und 3-Methyl-4-(4-trifluormethylthio-phenoxy)-anilin) werden in 1,2 l Methanol warm gelöst und unter Rühren portionsweise mit 1,8 g Natriumborhydrid versetzt. Die Reaktion wird dünnschichtchromatografisch überprüft. Es wird eingeengt, der Eindampfrückstand wird in Essigester gelöst, es wird mit 1 N Natronlauge und zweimal mit Wasser gewaschen, getrocknet und eingeengt. Der ölige Eindampfrückstand wird in wenig Ethanol gelöst, mit 6 ml ca. 5 N Lösung von Chlorwasserstoff in Ethanol versetzt und eingeengt. Beim Einengen tritt Kristallisation ein. Es wird mit Acetonitril verrührt, abgesaugt und mit Acetonitril gewaschen. Man erhält 5,0 g farblose Kristalle vom Schmelzpunkt ab 110°C unter Zersetzung.

Analog wurden hergestellt:

Beispiel 2

1-(4-Amino-3,5-dichlor-phenyl)-2-[3-methyl-4-(4-trifluormethylsulfonyl-phenoxy)-phenylamino]-ethanol-hydrochlorid vom Schmelzpunkt 143°C unter Zersetzung.

Beispiel 3

1-(4-Amino-3,5-dichlor-phenyl)-2-(4-n-butylthiophenyl-amino)-ethanol-hydrochlorid vom Schmelzpunkt 123-125°C unter Zersetzung.

Beispiel 4

1-(4-Amino-3,5-dichlor-phenyl)-2-[4-(4-chlorphenylthio)-3,5-dimethyl-phenylamino]-ethanol-hydrochlorid vom Schmelzpunkt 165°C.

Beispiel 5

1-(4-Amino-3,5-dichlor-phenyl)-2-(3-trifluor-propylamino)-ethanol

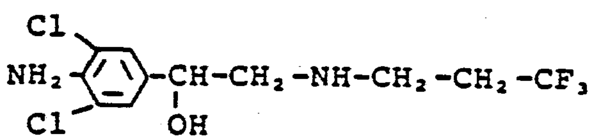

3,0 g 4-Amino-3,5-dichlor-ω-(3,3,3-trifluorpropylamino)-acetophenon (hergestellt aus 4-Amino-3,5-dichlor-ω-brom-acetophenon und 3-Trifluor-propylamin) werden in 40 ml

Methanol gelöst und unter Rühren bei 25 bis 35°C tropfenweise mit 0,96 g Natriumborhydrid in 4 ml Wasser versetzt. Die Reaktion wird dünnschichtchromatographisch überprüft. Es wird eingeengt, der Eindampfrückstand wird in Chloroform gelöst, es wird dreimal mit Wasser gewaschen, über Magnesiumsulfat getrocknet und eingeengt. Nach einmaligem Umkristallisieren des grobkristallinen Eindampfrückstandes aus Acetonitril erhält man 1,8 g hellrosa Kristalle vom Schmelzpunkt 115°C.

Analog wurden hergestellt:

Beispiel 6

1-(4-Amino-3,5-dichlor-phenyl)-2-(4-trifluor-butylamino)-ethanol vom Schmelzpunkt 104°C.

Beispiel 7

1-(4-Amino-3,5-dichlor-phenyl)-2-(1,3,3-trimethyl-6-aza-bicyclo[3.2.1]oct-6-yl)-1-ethanol vom Schmelzpunkt 129°C.

Beispiel 8

1-(4-Amino-3,5-dichlorphenyl)-2-(3-azabicyclo[3.2.2]non-3-yl)-1-ethanol vom Schmelzpunkt 151°C.

Beispiel 9

1-(4-Amino-3,5-dichlor-phenyl)-2-(3-azabicyclo[3.2.1]oct-3-yl)-1-ethanol vom Schmelzpunkt 135°C.

Beispiel 10

1-(4-Amino-3,5-dichlor-phenyl)-2-(3,3-dichlor-3-fluor-propylamino)-ethanol-hydrochlorid

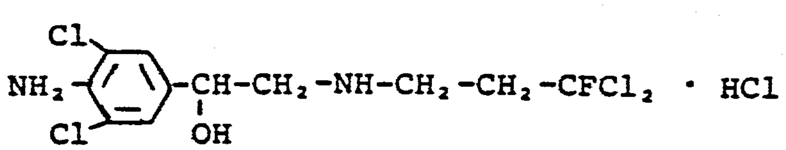

6,0 g 4-Amino-3,5-dichlor-ω-(3,3-dichlor-3-fluor-propylamino)-acetophenon (hergestellt aus 4-Amino-3,5-dichlor-ω-bromacetophenon und 3,3-Dichlor-3-fluor-propylamin) werden in 80 ml Methanol gelöst und unter Rühren bei 25 bis 35°C tropfenweise mit 1,8 g Natriumborhydrid in 8 ml Wasser versetzt. Die Reaktion wird dünnschichtchromatographisch überprüft.

Es wird eingeengt, der Eindampfrückstand wird in Chloroform gelöst, es wird einmal mit verdünnter Ammoniaklösung und zweimal mit Wasser gewaschen, über Magnesiumsulfat getrocknet und eingeengt. Der ölige Eindampfrückstand wird in Ether aufgenommen und mit einer 5 N Lösung von Chlorwasserstoff in Ethanol versetzt (bis pH 5). Nach beendeter Kristallisation wird abgesaugt und mit wenig kaltem Ethanol gewaschen. Nach einmaligem Umkristallisieren aus Isopropanol erhält man 2,0 g farblose Kristalle vom Schmelzpunkt 198°C.

Analog wurde hergestellt:

Beispiel 11

1-(4-Amino-3,5-dichlor-phenyl)-2-(2-difluorchlormethyl-propylamino)-ethanol-hydrochlorid vom Schmelzpunkt 183°C.

Beispiel 12

N-/2-(4-Amino-3,5-dichlor-phenyl)-2-(1,2-dimethylpropyl-dimethylsilyloxy)-ethyl/-N-t-butyl-N-(1,2-dimethylpropyl-dimethylsilyl)-amin

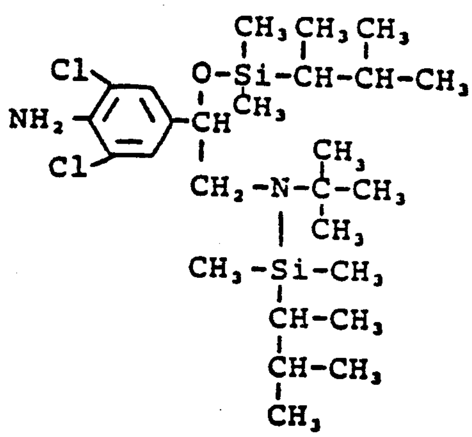

2,77 g 1-(4-Amino-3,5-dichlor-phenyl)-2-(1-butylamino)-ethanol (hergestellt aus 4-Amino-α-/(1-butylamino)-methyl/-3,5-dichlorbenzylalkohol und Natriumborhydrid) werden mit 2,04 g Imidazol und 3,64 g Dimethylisoamylsilylchlorid in 30 ml trockenem Dimethylformamid nach dem Abklingen der exothermen Reaktion noch 1 Stunde gerührt. Es wird im Hochvakuum eingeengt, der Eindampfrückstand wird in 50 ml Toluol gelöst, es wird dreimal

0119446

mit je 50 ml Wasser gewaschen, über Magnesiumsulfat getrocknet und eingeengt. Der ölige Eindampfrückstand wird in Petrolether gelöst und einen Tag bei 0°C stehengelassen. Spuren von nicht umgesetztem 1-(4-Amino-3,5-dichlorphenyl)-2-(1-butylamino)-ethanol werden abfiltriert, das klare Filtrat wird eingeengt und anschließend im Hochvakuum getrocknet. Man erhält 2,0 g dünnschichtchromatographisch reines hellgelbes Öl mit den gefundenen Analysenwerten:

57,3 C; 9,2 H; 5,2 N; 12,8 Cl; 10,6 % Si, gefundene Werte
58,5 C; 9,4 H; 5,3 N; 13,3 Cl; 10,5 % Si, berechnete Werte.

## Patentansprüche

1) Phenylethylamin-Derivate der allgemeinen Formel (I)

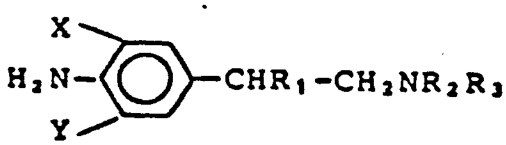

in der

| | |
|---|---|
| X und Y | gleich oder verschieden sind und für Wasserstoff oder Halogen stehen, |
| $R_1$ | für OH oder einen Rest 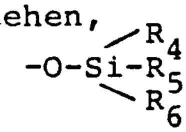 steht, |
| $R_2$ | Wasserstoff oder einen geradkettigen, verzweigten, gesättigten oder ungesättigten Alkylrest mit bis zu 6 C-Atomen bedeutet, mit der Maßgabe, daß $R_2$ nur dann für einen Alkylrest stehen kann, wenn $R_3$ $-SiR_4R_5R_6$ bedeutet, |
| $R_3$ | einen geradkettigen, verzweigten, gesättigten oder ungesättigten Alkylrest, der durch Halogen, bevorzugt Chlor und/oder Fluor substituiert ist, einen Rest $-SiR_4R_5R_6$, einen Phenylrest, der gegebenenfalls durch Halogen, Hydroxy, Alkyl, Aryl, Alkoxy, Alkylthio, gegebenenfalls substituiertes Phenoxy oder Phenylthio, Cyano oder Trifluormethyl substituiert sein kann, einen heterocyclischen Rest oder zusammen mit $R_2$ einen stickstoffhaltigen, gesättigten heterocyclischen Rest bedeutet und |

$R_4, R_5, R_6$ einen geradkettigen oder verzweigten Alkylrest bedeuten, und deren physiologisch verträglichen Salze.

2) Phenylethylamin-Derivate nach Anspruch 1, in denen

X und Y für Chlor stehen,

$R_1$ $O-SiR_4R_5R_6$,

$R_2$ einen gesättigten oder ungesättigten, verzweigten oder unverzweigten $C_1$-$C_6$-Alkylrest und

$R_3$ den Rest $SiR_4R_5R_6$ bedeutet,

sowie deren physiologisch verträglichen Salze.

3) Phenylethylamin-Derivate nach Anspruch 1, in denen

X und Y Chlor,

$R_1$ OH,

$R_2$ Wasserstoff und

$R_3$ entweder einen durch ein oder mehrere Chlor- oder Fluoratome substituierten geradkettigen oder verzweigten, gesättigten oder ungesättigten $C_1$-$C_6$-Alkylrest oder einen Phenylrest, der gegebenenfalls durch Halogen, Hydroxy, $C_1$-$C_4$-Alkyl, $C_1$-$C_4$-Alkoxy, $C_6$-$C_{10}$-Aryl, $C_1$-$C_4$-Alkylthio oder durch gegebenenfalls substituiertes Phenoxy oder Phenylthio substituiert sein kann, bedeutet,

sowie deren physiologisch verträglichen Salze.

4) Phenylethylamin-Derivate nach Anspruch 1, in denen

X und Y für Chlor,
$R_1$ für OH,
$R_2$ für Wasserstoff und
$R_3$ für eine durch ein oder mehrere $CF_3S$-, $CF_3SO_2$-, $C_1$-$C_4$-Alkyl-, Halogen- oder Phenyl-Reste substituierte Phenoxyphenyl- oder Phenylthiophenyl-Gruppe steht,

sowie deren physiologisch verträglichen Salze.

5) Verfahren zur Herstellung der Phenylethylamin-Derivate der Formel (I) nach Anspruch 1, dadurch gekennzeichnet, daß man

a) Verbindungen der allgemeinen Formel (II)

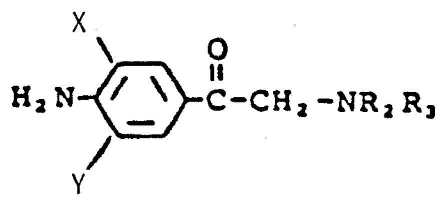

in der
X,Y,$R_2$ und $R_3$ die oben angegebene Bedeutung haben,

reduziert oder

b) zur Herstellung der Verbindungen der Formel (I), in denen

$R_1$ für den Rest $-O-SiR_4R_5R_6$, in dem $R_4$, $R_5$, $R_6$ einen geradkettigen oder verzweigten Alkylrest bedeuten,

$R_2$ für einen geradkettigen, verzweigten, gesättigten oder ungesättigten Alkylrest mit bis zu 6 C-Atomen und

$R_3$ für den Rest $-SiR_4R_5R_6$ steht,

Verbindungen der allgemeinen Formel (III)

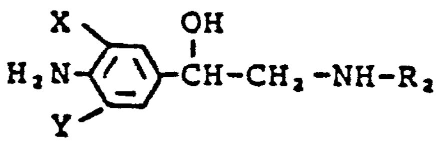

in der

X,Y für Chlor stehen und $R_2$ die oben angegebene Bedeutung hat,

mit Silylierungsmitteln der Formel (IV)

$$R_4R_5R_6\ Si-Z$$

in der

Z Halogen, $-O-SO_2-CF_3$, $-O-SiR_4R_5R_6$ oder $-O-SO_2-O-SiR_4R_5R_6$ bedeutet,

umsetzt.

6) Verwendung von Phenylethylamin-Derivaten der allgemeinen Formel (I)

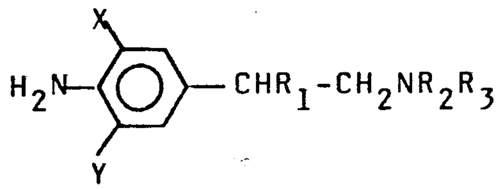

in der

X und Y gleich oder verschieden sind und für Wasserstoff oder Halogen stehen,

$R_1$ für OH oder einen Rest 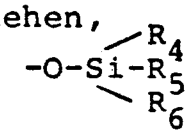 steht,

$R_2$ Wasserstoff oder einen geradkettigen, verzweigten, gesättigten oder ungesättigten Alkylrest mit bis zu 6 C-Atomen bedeutet, mit der Maßgabe, daß $R_2$ nur dann für einen Alkylrest stehen kann, wenn $R_3$ $-SiR_4R_5R_6$ bedeutet,

$R_3$ einen geradkettigen, verzweigten, gesättigten oder ungesättigten Alkylrest, der durch Halogen, bevorzugt Chlor und/oder Fluor substituiert ist, einen Rest $-SiR_4R_5R_6$, einen Phenylrest, der gegebenenfalls durch Halogen, Hydroxy, Alkyl, Aryl, Alkoxy, Alkylthio, gegebenenfalls substituiertes Phenoxy oder Phenylthio, Cyano oder Trifluormethyl substituiert sein kann, einen heterocyclischen Rest oder zusammen mit $R_2$ einen stickstoffhaltigen, gesättigten heterocyclischen Rest bedeutet und

$R_4$, $R_5$, $R_6$ einen geradkettigen oder verzweigten Alkylrest bedeuten,

und deren physiologisch verträglichen Salze als Wachstumsförderer für Tiere.

7) Verwendung von Phenylethylamin-Derivaten nach Anspruch 1, in denen

X und Y für Chlor stehen,
$R_1$ $O-SiR_4R_5R_6$,
$R_2$ einen gesättigten oder ungesättigten, verzweigten oder unverzweigten $C_1-C_6$-Alkylrest und
$R_3$ den Rest $-SiR_4R_5R_6$ bedeutet,

sowie deren physiologisch verträglichen Salze.

8) Verwendung von Phenylethylamin-Derivaten nach Anspruch 1, in denen

X und Y für Chlor,
$R_1$ für OH,
$R_2$ für Wasserstoff und
$R_3$ für eine durch ein oder mehrere $CF_3S$-, $CF_3SO_2$-, $C_1-C_4$-Alkyl-, Halogen- oder Phenyl-Reste substituierte Phenoxyphenyl- oder Phenylthiophenyl-Gruppe steht,

sowie deren physiologisch verträglichen Salze als Wachstumsförderer für Tiere.

9) Tiernahrung enthaltend Phenylethylamin-Derivate der allgemeinen Formel (I) von Anspruch 1.

10) Praemixe für die Tiernahrung, enthaltend Phenylethylamin-Derivate der allgemeinen Formel (I) von Anspruch 1.